# EUROPEAN PATENT APPLICATION

(11) **EP 3 572 075 A2**
(43) Date of publication of application: **27.11.2019**
(21) Application number: 18741071.7
(22) Date of filing: 23.01.2018
(51) Int. Cl.: A61K 9/24, A61K 9/20, A61K 31/4365, A61K 31/505, A61K 45/06

(54) **COMPLEX FORMULATION COMPRISING HMG-COA REDUCTASE INHIBITOR AND CLOPIDOGREL**

(30) Priority: 23.01.2017 KR 20170010532
(71) Applicant: Dong Wha Pharm. Co., Ltd., Jung-gu Seoul 04522 (KR)
(72) Inventor: BAEK, Nam Hyun, Anyang-si Gyeonggi-do 14039 (KR); CHOI, Min Soo, Suwon-si Gyeonggi-do 16709 (KR); KIM, Ja Young, Yongin-si Gyeonggi-do 17084 (KR); WOO, Ji Young, Hwaseong-si Gyeonggi-do 18442 (KR); SEONG, Seung Kyoo, Anyang-si Gyeonggi-do 14046 (KR); SONG, Young Jun, Seoul 07979 (KR); SEO, Ki Soo, Suwon-si Gyeonggi-do 16695 (KR); HEO, Woon, Yongin-si Gyeonggi-do 17067 (KR); JUNG, Taeseong, Yongin-si Gyeonggi-do 17081 (KR)
(74) Representative: Gille Hrabal
(86) International application number: PCT/KR2018/001033
(87) International publication number: WO 2018/135932

(57) **Abstract**

The present invention relates to a complex preparation comprising clopidogrel, an HMG-CoA reductase inhibitor, and a separation membrane containing a hydrophobic compound. More particularly, an objective of the present invention is to provide the complex preparation comprising clopidogrel and the HMG-CoA reductase inhibitor, wherein the complex preparation is intended for preventing or treating a cardiovascular disease, which has excellent storage stability by preventing a decrease in the stability of the HMG-CoA reductase inhibitor.

## Description

### Technical Field

The present invention relates to a complex preparation including clopidogrel, an HMG-CoA reductase inhibitor, and a separation membrane containing a hydrophobic compound. Particularly, the present invention relates to a complex preparation for preventing or treating a cardiovascular disease, including clopidogrel and an HMG-CoA reductase inhibitor, which has excellent storage stability by using a separation membrane containing a hydrophobic compound.

### Background

An HMG-CoA reductase inhibitor has been used as a drug for treating hyperlipidemia for several decades. Active components thereof have an effect of lowering total cholesterol and low density lipoprotein (LDL) cholesterol and raising a high density lipoprotein (HDL) cholesterol level in a human body. The HMG-CoA reductase inhibitor exerts a remedial effect in such a way that this inhibitor inhibits an activity of HMG-CoA reductase in an early stage of converting HMG-CoA into mevalonate during a cholesterol biosynthesis, thereby lowering a production of cholesterol, and that in compensation for such loss, this inhibitor increases the number of LDL receptors and thus brings a more number of LDLs from blood, thereby lowering an LDL concentration in blood. As the HMG-CoA reductase inhibitor, there are simvastatin (U.S. Patent No. 4,448,784), lovastatin (U.S. Patent No. 4,231,938), mevastatin (U.S. Patent No. 3,983,140), pravastatin (U.S. Patent No. 4,450,171), fluvastatin (U.S. Patent No. 5,260,440), cerivastatin (U.S. Patent No. 6,218,403), atorvastatin (U.S. Patent No. 5,627,176), rosuvastatin (U.S. Patent No. 7,842,807), pitavastatin (U.S. Patent No. 5,856,336), bervastatin (U.S. Patent No. 5,082,859), dalvastatin (U.S. Patent No. 4,863,957), glenvastatin (U.S. Patent No. 4,925,852), etc.

The HMG-CoA reductase inhibitor has a problem in that this inhibitor is easily decomposed in a certain environment, in particular, in an acidic environment. In result, the HMG-CoA reductase inhibitor is not easily formulated into a complex preparation with other acidic active components or acidic salt substitutes, thus lowering the stability of being produced into and stored as a complex preparation. As a representative acid decomposition product, there is (3R, 5S) lactone (hereinafter, referred to as "lactone"), and said lactone is produced in such a way that a hydroxyl group adjacent to a carbon-carbon double bond is oxidized into a ketone functional group in an acidic environment.

Meanwhile, as a mechanism for causing arteriosclerosis, there is thrombogenesis. A blood clot is formed in an injured blood vessel by means of an interaction between blood platelets and plasma coagulation factors, thus causing arteriosclerosis.

Clopidogrel(5-methyl-α-(4,5,6,7,-tetrahydro[2,3-c]thienopyridyl)(2-chlorophenyl)acetate) (U.S. Patent No. 6,504,030) directly inhibits adenosine diphosphate (hereinafter, referred to as "ADP") from binding to an ADP receptor, which is known to play an important role in thrombogenesis, and directly inhibits the ADP activation of a glucoprotein GPIIb/IIa complex, thus specifically inhibiting a platelet agglutination. Thus, clopidogrel is used in preventing and treating thromboembolism such as stroke or myocardial infarction.

A clopidogrel free base is an oily component, which has a low solubility and is hard to be formulated into a solid preparation, and thus this free base is difficult to maintain high purity and has low stability. In result, clopidogrel commonly used in a pharmaceutical preparation is used in such a form that is substituted with an acid salt such as hydrogensulfate, hydrochloride, napadisilate, besylate, acetate, naphthalenesulfonate, etc. As a representative acid salt, clopidogrel hydrogensulfate is prepared by using a strong acid, which is concentrated during a preparation process, for substitution with the acid salt. Due to an acidic substance used at that time, clopidogrel hydrogensulfate is characterized by having a strong acidity. When preparing a complex preparation, such acidic property has a negative influence on the stability of a complex preparation due to its interaction with other active components, thus making it difficult to formulate the complex preparation.

A concurrent intake of the HMG-CoA reductase inhibitor and clopidogrel may achieve respective drug effects at the same time, such that a cardiovascular disease of various causes may be effectively treated; a convenience for intake may be increased more than an individual preparation; and a synergy effect may be expected due to a combined administration. However, the HMG-CoA reductase inhibitor is characterized by having stability thereof drastically decreased in an acidic environment, and thus it has a limit on being formulated into a complex preparation with clopidogrel.

Thus, the complex preparation of clopidogrel and the HMG-CoA reductase inhibitor requires a special type of dosage form so as to enhance stability thereof.

### Prior Art References

### Patent Documents

(Patent Document 1) U.S. Patent No. 4,448,784
(Patent Document 2) U.S. Patent No. 4,231,938
(Patent Document 3) U.S. Patent No. 3,983,140
(Patent Document 4) U.S. Patent No. 4,450,171
(Patent Document 5) U.S. Patent No. 5,260,440
(Patent Document 6) U.S. Patent No. 6,218,403
(Patent Document 7) U.S. Patent No. 5,627,176
(Patent Document 8) U.S. Patent No. 7,842,807
(Patent Document 9) U.S. Patent No. 5,856,336
(Patent Document 10) U.S. Patent No. 6,504,030
(Patent Document 11) U.S. Patent No. 5,082,859
(Patent Document 12) U.S. Patent No. 4,863,957
(Patent Document 13) U.S. Patent No. 4,925,852

### Detailed Description of the Invention

### Technical Problem

An objective of the present invention is to provide a complex preparation including clopidogrel and an HMG-CoA reductase inhibitor as an effective component, and including a separation membrane containing a hydrophobic compound.

Other objective of the present invention is to provide a method for preparing a complex preparation including clopidogrel and an HMG-CoA reductase inhibitor as an effective component, and including a separation membrane containing a hydrophobic compound.

### Technical Solution

The present invention provides a complex preparation including a pharmaceutically acceptable acid salt of clopidogrel, optical isomers thereof, hydrates or solvates thereof, or mixtures thereof; an HMG-CoA reductase inhibitor, pharmaceutically acceptable salts thereof, optical isomers thereof, hydrates or solvates thereof, or mixtures thereof; and a separation membrane containing a hydrophobic compound.

In the complex preparation of the present invention, said pharmaceutically acceptable acid salt of clopidogrel, optical isomers thereof, hydrates or solvates thereof, or mixtures thereof; and the HMG-CoA reductase inhibitor, pharmaceutically acceptable salts thereof, optical isomers thereof, hydrates or solvates thereof, or mixtures thereof may be physically separated from each other by means of said separation membrane and may not be in contact with each other.

Said complex preparation according to the present invention has excellent storage stability by preventing a decrease in the stability of HMG-CoA caused by a proton (H+) derived from the clopidogrel acid salt, and may be used as a valuable complex preparation for a use in preventing or treating a cardiovascular disease.

The complex preparation of the present invention involves a first active component compartment including a first active component; a separation membrane containing a hydrophobic compound; and a second active component compartment including a second active component.

Said separation membrane separates said first active component compartment and second active component compartment and thus prevents said first active component and second active component from coming in contact with each other, such that this separation membrane may perform a role in inhibiting a decrease in the stability of the HMG-CoA reductase inhibitor caused by the acid salt of clopidogrel.

Said first and second active components are different from each other, and said first and second active components may be one of i) a pharmaceutically acceptable acid salt of clopidogrel, optical isomers thereof, hydrates or solvates thereof, or mixtures thereof, or ii) an HMG-CoA reductase inhibitor, pharmaceutically acceptable salts thereof, optical isomers thereof, hydrates or solvates thereof, or mixtures thereof.

The complex preparation of the present invention relates to a complex preparation, in which it involves: a first active component-containing layer including a first active component; a separation membrane layer containing a hydrophobic compound; and a second active component-containing layer containing a second active component, in which said first and second active components are different from each other, and in which said first and second active components are one of i) a pharmaceutically acceptable acid salt of clopidogrel, optical isomers thereof, hydrates or solvates thereof, or mixtures thereof; or ii) an HMG-CoA reductase inhibitor, pharmaceutically acceptable salts thereof, optical isomers thereof, hydrates or solvates thereof, or mixtures thereof.

According to exemplary embodiments of the present invention, the present invention may be a complex preparation including a first active component-containing layer; a second active component-containing layer; and a separation membrane layer containing a hydrophobic separation membrane present between said first active component-containing layer and said second active component-containing layer. Particularly, the present invention involves: a) a first layer including a first active component; b) a second layer formed on said first layer and including a separation membrane containing a hydrophobic compound; and c) a third layer including a second active component, in which the first active component and second active component of said first layer and third layer may be different from each other.

In the complex preparation of the present invention, said first active component and second active component may be physically separated by means of said separation membrane and thus may not be in contact with each other. In the present invention, "being physically separated" refers to a state in which a plurality of active components are separated from each other so as not to cause any interaction with each other while a preparation thereof is stored. The complex preparation of the present invention has excellent storage stability because each active component is physically separated to prevent a decrease in the stability of HMG-CoA caused by a proton (H+) derived from a clopidogrel acid salt.

The complex preparation of the present invention may further include a coating layer between said active component layer and separation membrane layer. For example, said coating layer may be formed on one of the first or second active component layer, and may be formed on both of the first and second active component layers. Said additionally included coating layer may have a coating base generally used in the art, and particularly may include one or more substances from povidone, copovidone, methyl cellulose, hydroxymethyl cellulose, hydroxyethyl cellulose, hydroxypropyl cellulose, hydroxypropyl methylcellulose, hydroxypropyl ethylcellulose, gelatin and gum, but not limited thereto.

Said first and second active components may be one of i) a pharmaceutically acceptable acid salt of clopidogrel, optical isomers thereof, hydrates or solvates thereof, or mixtures thereof; or ii) an HMG-CoA reductase inhibitor, pharmaceutically acceptable salts thereof, optical isomers thereof, hydrates or solvates thereof, or mixtures thereof.

The complex preparation of the present invention may be a tablet consisting of: a first layer including a first active component in a plain tablet form; a second layer of a separation membrane containing a hydrophobic compound coated on said first layer; and a third layer including a second active component coated on said separation membrane layer, in which said first layer may take on such a form that the first active component and a pharmaceutically acceptable carrier are mixed.

In exemplary embodiments of the present invention, said first layer in the plain tablet form may involve: an inactive core including a pharmaceutically acceptable carrier; and a first active component-containing layer formed on said inactive core.

In other exemplary embodiments of the present invention, said first layer in the plain tablet form may be the one formed in such a way that the pharmaceutically acceptable carrier is mixed with the first active component.

According to exemplary embodiments of the present invention, said tablet may be a multi-layered tablet.

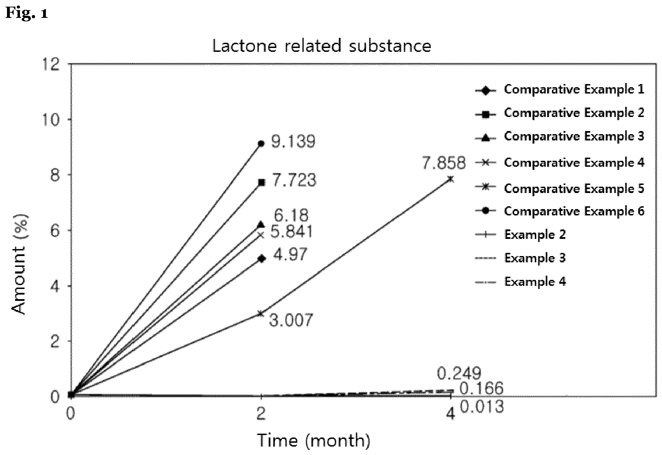

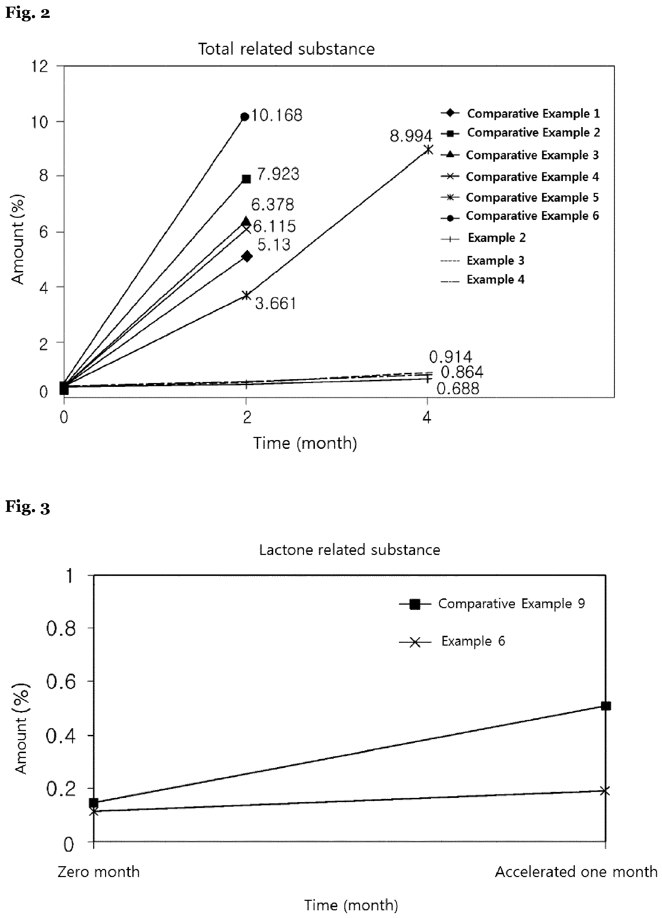

According to other exemplary embodiments of the present invention, the tablet of the present invention, including a separation membrane containing a hydrophobic compound, has very excellent storage stability because this tablet has a very low amount of lactone related substances and total related substances, which are acid decomposition products of the HMG-CoA reductase inhibitor (Table 7 and Figs. 1 and 2).

In the present invention, said pharmaceutically acceptable carrier refers to a pharmaceutically acceptable excipient, binder, disintegrant and lubricant, which are generally used in the art. For example, such excipient may include one or more substances from lactose, microcrystalline cellulose, silicified microcrystalline cellulose, silicic acid anhydride, calcium phosphate, dextrin, dextrose, dextrate, mannitol, maltose, sorbitol, sucrose, or mixtures thereof; such binder may include one or more substances from povidone, copovidone, methyl cellulose, hydroxymethyl cellulose, hydroxypropyl cellulose, hydroxyethyl cellulose, gelatin, gum, or mixtures thereof; such disintegrant may include one or more substances from crospovidone, croscarmellose sodium, sodium starch glycolate, pre-gelatinized starch, grain starch, or mixtures thereof; and such lubricant may include one or more substances from magnesium stearate, stearic acid, talc, sodium stearyl fumarate, sodium lauryl sulfate, poloxamer, or mixtures thereof, but not limited thereto.

Particularly, the complex preparation of the present invention may involve: an inactive core including a pharmaceutically acceptable carrier; a first active component-containing layer formed on said inactive core; a separation membrane layer containing a hydrophobic compound formed on said first active component-containing layer; and a second active component-containing layer including a second active component formed on said separation membrane layer, and this complex preparation may take on such a form that said inactive core and first active component are mixed together.

More particularly, if the first active component is a pharmaceutically acceptable acid salt of clopidogrel, optical isomers thereof, hydrates or solvates thereof, or mixtures thereof, the second active component may be an HMG-CoA reductase inhibitor, pharmaceutically acceptable salts thereof, optical isomers thereof, hydrates or solvates thereof, or mixtures thereof. Also, if the first active component is the HMG-CoA reductase inhibitor, pharmaceutically acceptable salts thereof, optical isomers thereof, hydrates or solvates thereof, or mixtures thereof, the second active component may be the pharmaceutically acceptable acid salt of clopidogrel, optical isomers thereof, hydrates or solvates thereof, or mixtures thereof. For example, said first active component may be an acid salt of clopidogrel, and the second active component is the HMG-CoA reductase inhibitor or pharmaceutically acceptable salts thereof, which may be rosuvastatin or pharmaceutically acceptable salts thereof.

The complex preparation of the present invention relates to a complex preparation, in which it involves: a first particle including a first active component; a separation membrane containing a hydrophobic compound; and a second particle including a second active component, in which said first and second active components are different from each other, and in which said first and second active components are one of i) a pharmaceutically acceptable acid salt of clopidogrel, optical isomers thereof, hydrates or solvates thereof, or mixtures thereof; or ii) an HMG-CoA reductase inhibitor, pharmaceutically acceptable salts thereof, optical isomers thereof, hydrates or solvates thereof, or mixtures thereof.

In the present invention, said separation membrane containing the hydrophobic substance separates said first particle and second particle and thus may perform a role in preventing said first active component and second active component from being in contact with and penetrating into each other.

In the present invention, said first and second particles may take on a form of powder, pellet, granule, tablet or the like, which may take on a particle form, equal to or different from each other. For example, said first and second particles may be all powder, all pellet, all granule or all tablet, and one may be the pellet, and the other may be the granule, powder or tablet, but not limited thereto, and may be appropriately adjusted, if necessary.

According to exemplary embodiments of the present invention, the complex preparation may be a capsule preparation: involving a first particle including a first active component; a separation membrane containing a hydrophobic compound; and a second particle including a second active component.

Said capsule preparation includes the separation membrane, thereby not causing a phenomenon, in which the first particle and the second particle are mixed with each other, and thus the first particle and the second particle may be present in a state of being separated from each other within the capsule. Said separation membrane may be formed on one of the first or second particle, and may be formed on both of the first and second particles.

In exemplary embodiments of the present invention, said particle may be a pellet, and said pellet may be the one formed in such a way that an active component is coated on an inactive pellet.

Particularly, the complex preparation of the present invention may be the capsule preparation involving: an inactive pellet including a pharmaceutically acceptable carrier; a first particle including a first active component formed on said pellet; a separation membrane containing a hydrophobic compound formed on said first particle; and a second particle physically separated from the first particle and including a second active component. In such case, said second particle may be a powder itself of said second active component or the second particle may include the second active component and the pharmaceutically acceptable carrier.

According to exemplary embodiments of the present invention, said capsule preparation may further include the pharmaceutically acceptable carrier along with the first and second particles. Said pharmaceutically acceptable carrier refers to a pharmaceutically acceptable excipient, binder, disintegrant and lubricant, which are generally used in the art, and specific examples thereof are as described above.

Also, the complex preparation of the present invention may be the one involving: a first particle including a first active component formed on an inactive pellet including a pharmaceutically acceptable carrier; a second particle including a second active component formed on an inactive pellet including a pharmaceutically acceptable carrier; and a separation membrane formed on one or more of said first or second particle, in which said separation membrane may be formed on both of the first and second particles or may be formed on only one selected from the first and second particles.

Further, the complex preparation of the present invention may include a first active component, a second active component and a particle including a separation membrane, which prevents said first active component and said second active component from being in contact with each other and includes a hydrophobic compound. Particularly, the complex preparation of the present invention may involve a first layer including said first active component; a second layer including said second active component; and a particle including a separation membrane present between said first layer and said second layer. More particularly, the complex preparation of the present invention may be a capsule preparation involving: a first layer including a first active component formed on an inactive pellet including a pharmaceutically acceptable carrier; a second layer including a second active component formed on an inactive pellet including a pharmaceutically acceptable carrier; and a particle including a separation membrane present between said first layer and second layer.

More particularly, if the first active component is a pharmaceutically acceptable acid salt of clopidogrel, optical isomers thereof, hydrates or solvates thereof, or mixtures thereof, the second active component may be an HMG-CoA reductase inhibitor, pharmaceutically acceptable salts thereof, optical isomers thereof, hydrates or solvates thereof, or mixtures thereof. Also, if the first active component is the HMG-CoA reductase inhibitor, pharmaceutically acceptable salts thereof, optical isomers thereof, hydrates or solvates thereof, or mixtures thereof, the second active component may be the pharmaceutically acceptable acid salt of clopidogrel, optical isomers thereof, hydrates or solvates thereof, or mixtures thereof. For example, said first active component may be the acid salt of clopidogrel, and the second active component may be the HMG-CoA reductase inhibitor or pharmaceutically acceptable salts thereof, which may be rosuvastatin or pharmaceutically acceptable salts thereof.

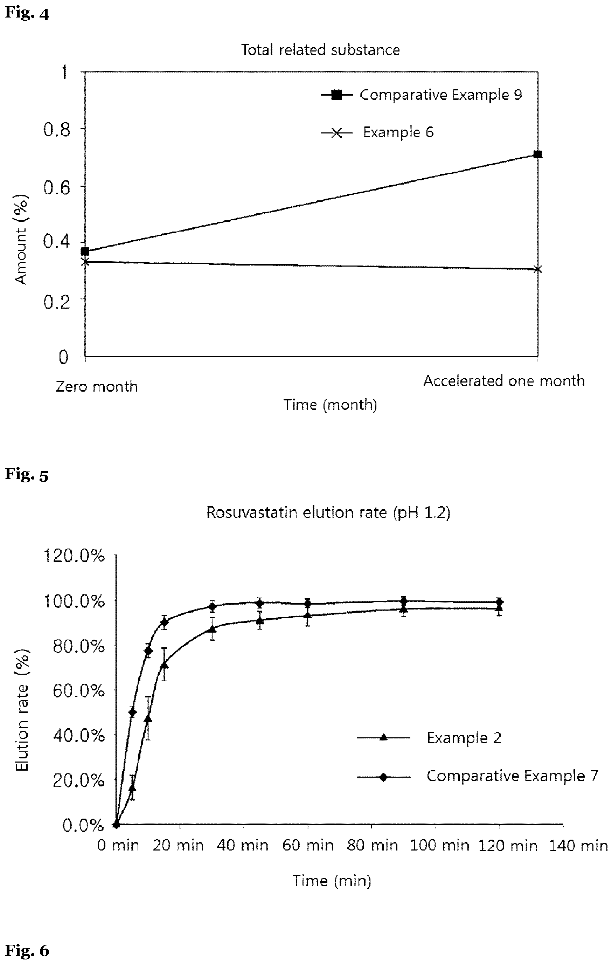

According to exemplary embodiments of the present invention, the capsule preparation of the present invention including a separation membrane containing a hydrophobic compound has very excellent storage stability because this capsule preparation has a very low amount of lactone related substances and total related substances, which are acid decomposition products of the HMG-CoA reductase inhibitor (Table 8 and Figs. 3 and 4).

In the present invention, said first particle and second particle may include one or more pharmaceutically acceptable carriers, and said carrier may be mixed with the first and second particles or may be present as a core of the first and second active components. A type of said carrier is not particularly limited, and may be the same as or different from each other. For example, the carrier refers to a pharmaceutically acceptable excipient, binder, disintegrant and lubricant. Particularly, such excipient may be one or more substances from lactose, microcrystalline cellulose, silicified microcrystalline cellulose, silicic acid anhydride, calcium phosphate, dextrin, dextrose, dextrate, mannitol, maltose, sorbitol, sucrose, or mixtures thereof; such binder may be one or more substances from povidone, copovidone, methyl cellulose, hydroxymethyl cellulose, hydroxypropyl cellulose, hydroxyethyl cellulose, gelatin, gum, or mixtures thereof; such disintegrant may be one or more substances from crospovidone, croscarmellose sodium, sodium starch glycolate, pre-gelatinized starch, grain starch, or mixtures thereof; such lubricant may be one or more substances from magnesium stearate, stearic acid, talc, sodium stearyl fumarate, sodium lauryl sulfate, poloxamer, or mixtures thereof, but not limited thereto.

Clopidogrel, which is a pharmacologically active component used in the present invention, is a drug used in preventing and treating a cardiovascular disease such as a stroke, myocardial infarction, arteriosclerosis, etc., and clopidogrel also specifically inhibits a platelet agglutination by directly inhibiting ADP from binding to an ADP receptor, and directly inhibiting an ADP activation of a glucoprotein GPIIb/IIa complex.

Clopidogrel, which may be used as an effective component of the present invention, refers to a pharmaceutically acceptable acid salt of clopidogrel, optical isomers thereof, hydrates or solvates thereof, or mixtures thereof, and particularly may be at least one selected from the group consisting of acid salts of clopidogrel hydrogensulfate, hydrochloride, napadisilate, besylate, bromate, taurocholate or acetate, optical isomers thereof and hydrates or solvates thereof, but not limited thereto.

According to the present invention, said pharmaceutically acceptable acid salt of clopidogrel, optical isomers thereof, hydrates or solvates thereof, or mixtures thereof may be included as clopidogrel in an amount of 75 to 300 mg per preparation, and may be used by being prepared in a pharmaceutically acceptable form of granule, pellet, tablet, film, etc.

An HMG-CoA reductase inhibitor of the present invention is a drug for treating and preventing hyperlipidemia and arteriosclerosis by lowering a concentration of lipoprotein or lipid in blood. According to the present invention, the HMG-CoA reductase inhibitor refers to the HMG-CoA reductase inhibitor, pharmaceutically acceptable salts thereof, optical isomers thereof, hydrates or solvates thereof, or mixtures thereof, and particularly may be at least one selected from the group consisting of rosuvastatin, atorvastatin, lovastatin, pravastatin, simvastatin, mevastatin, lavastatin, fluvastatin, cerivastatin, pitavastatin, bervastatin, dalvastatin, glenvastatin, pharmaceutically acceptable salts thereof, optical isomers thereof, hydrates or solvates thereof, and mixtures thereof, and more particularly may be rosuvastatin, atorvastatin, pravastatin, fluvastatin, pharmaceutically acceptable salts thereof, optical isomers thereof, hydrates or solvates thereof, or mixtures thereof, but not limited thereto.

According to the present invention, said HMG-CoA reductase inhibitor, pharmaceutically acceptable salts thereof, optical isomers thereof, hydrates or solvates thereof, or mixtures thereof may be included in a content, which is generally usable as a therapeutic agent for hyperlipidemia; may be included as an active component, for example, in an amount of 1 to 80 mg; and may be used by being prepared in a pharmaceutically usable form of granule, pellet, tablet, film, etc.

A separation membrane containing a hydrophobic compound of the present invention prevents or minimizes an effect of an acidic substance such as a proton, etc., derived from an acid salt of clopidogrel on the HMG-CoA reductase inhibitor. Said separation membrane is a pH-independent membrane unlike a pH-dependent enteric separation membrane, in which pH does not influence a performance of the separation membrane. Particularly, said separation membrane may be easily dissolved or decomposed in an acidic environment. Thus, said separation membrane containing the hydrophobic compound inhibits an acidic substance derived from an acid salt of clopidogrel from interacting with the HMG-CoA reductase inhibitor and lowering stability, and also enables clopidogrel and the HMG-CoA reductase inhibitor to be easily disintegrated and eluted in a gastrointestinal tract at the same time without an influence of pH, when being administered in vivo.

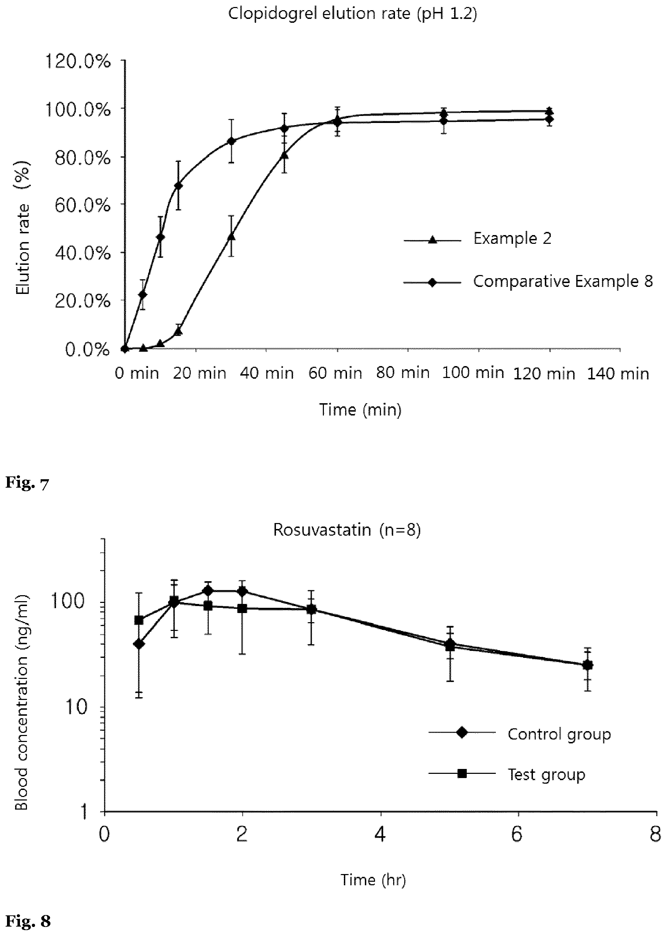

According to exemplary embodiments of the present invention, the inventive complex preparation including the separation membrane containing the hydrophobic compound is disintegrated within a short period of time (Table 6) and enables active components, i.e., clopidogrel and the HMG-CoA reductase inhibitor to be eluted within a short period of time under a pH condition similar to the gastrointestinal tract (Table 9 and Figs. 5 and 6).

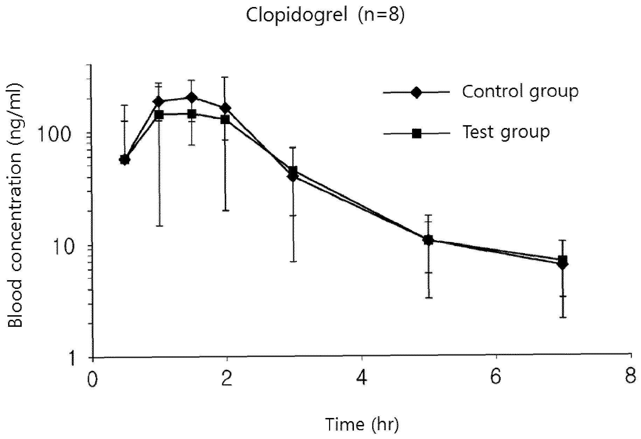

Also, according to other exemplary embodiments of the present invention, the inventive complex preparation including the separation membrane containing the hydrophobic compound does not have any significant difference in PK parameters in a body compared to a commercially available single preparation of an active component (Comparative Examples 7 and 8), and thus may be utilized as an excellent therapeutic agent for a cardiovascular disease (Table 11 and Figs. 7 and 8).

The separation membrane included in the complex preparation of the present invention has a hydrophobic compound. Particularly, said separation membrane may further include a hydrophilic compound in addition to the hydrophobic compound, and said separation membrane may include said hydrophobic compound and hydrophilic compound in a weight ratio of 1: 10 to 3 : 1, and more particularly in a weight ratio of 1: 2 to 2 : 1.

In the present invention, said hydrophobic compound may be included in an amount of about 0.01 to 0.8 parts by weight, particularly in an amount of about 0.1 to 0.5 parts by weight per 1 part by weight of the separation membrane. If the hydrophobic compound is less than 0.01 parts by weight, an acidic substance derived from an acid salt of clopidogrel interacts with the HMG-CoA reductase inhibitor and thus causes a concern about a decrease in stability. If the hydrophobic compound is more than 0.8 parts by weight, the separation membrane is not smoothly disintegrated and dissolved, and thus a drug release does not occur or is excessively delayed, such that such release may be also greatly delayed in a body.

In the present invention, said hydrophobic compound is the one, which plays a role in inhibiting an acidic substance such as a proton (H+), etc., derived from an acid salt of clopidogrel from interacting with the HMG-CoA reductase inhibitor; refers to a substance, which is not dissolved at all or dissolved in a very little amount in water, for example, the substance, which requires 1000 ml or more of water to dissolve 1 g or 1 ml of the hydrophobic compound; and also refers to a pharmaceutically acceptable substance. Particularly, said hydrophobic compound may be one or more compounds selected from the group consisting of fatty acid and fatty acid esters, fatty acid alcohols, waxes and mixtures thereof. More particularly, the hydrophobic compound may be fatty acid esters, fatty acid alcohols, waxes or mixtures thereof, more particularly fatty acid esters, waxes or mixtures thereof. For example, said hydrophobic compound may be: fatty acid and fatty acid esters such as glyceryl palmitostearate, glyceryl stearate, glyceryl behenate, cetyl palmitate, lecithin, glyceryl monooleate or stearic acid; fatty acid alcohols such as cetostearyl alcohol, cetyl alcohol, stearyl alcohol or the like; waxes such as carnauba wax, cera, microcrystalline wax, etc.; or mixtures thereof, and particularly may be glyceryl behenate, sodium stearyl fumarate, carnauba wax, lecithin or mixtures thereof, but not limited thereto.

In the present invention, said hydrophilic compound is the one, which plays a role in facilitating an attachment of a hydrophobic compound, and refers to a water-soluble compound and pharmaceutically acceptable substance, which controls (adjusts) a drug release. Said hydrophilic compound may be included in an amount of about 0.2 to 0.99 parts by weight, particularly in an amount of about 0.5 to 0.9 parts by weight per 1 part by weight of the separation membrane. The hydrophilic compound of the present invention may be, for example, a cellulose derivative, gums, proteins, polyvinyl derivative, polymethacrylate copolymer, polyethylene derivative, carboxyvinyl polymer or mixtures thereof. Particularly, said hydrophilic compound may be the cellulose derivative, polyvinyl derivative, carboxyvinyl polymer, polyethylene derivative or mixtures thereof, more particularly hydroxypropyl methylcellulose, polyvinyl pyrrolidone, copolyvidone, carbomer, polyethylene oxide or mixtures thereof, and much more particularly copovidone, but not limited thereto. For example, said hydrophilic compound may be the cellulose derivative such as hydroxypropyl methylcellulose, hydroxypropyl cellulose, hydroxymethyl cellulose, hydroxyethyl cellulose, methyl cellulose, sodium carboxymethyl cellulose, hydroxypropyl methylcellulose acetate succinate, hydroxyethyl methylcellulose or the like; gums such as guar gum, locust bean gum, tragacanth, carrageenan, acacia gum, arabic gum, gellan gum, xanthan gum or the like; proteins such as gelatin, casein, zein or the like; the polyvinyl derivative such as polyvinyl alcohol, polyvinyl pyrrolidone, copolyvidone, polyvinyl acetal dietylamino acetate or the like; the polymethacrylate copolymer such as poly(butylmethacrylate-(2-dimethylaminoethyl)methacrylate-methyl methacrylate) copolymer, poly(methacrylic acid-methyl methacrylate) copolymer, poly(methacrylic acid-ethyl acrylate) copolymer or the like; the polyethylene derivative such as polyethylene glycol, polyethylene oxide or the like; the carbomer as carboxy vinyl polymer; and mixtures thereof, but not limited thereto.

The complex preparation of the present invention may further include a pharmaceutically acceptable additive, if necessary. For example, such complex preparation may be formulated into a preparation by including an additive such as a pharmaceutically acceptable stabilizer, binder, disintegrant, lubricant, diluent, coating agent, pH-adjusting agent, dissolution aid, surfactant, etc., within the range that does not undermine an effect of the present invention.

The complex preparation according to the present invention may further include the stabilizer to improve the stability of the HMG-CoA reductase inhibitor, and said stabilizer is a component capable of stabilizing the HMG-CoA reductase inhibitor, which is unstable to acid, in which a pharmaceutically acceptable substance conventionally used in the art may be used. For example, said stabilizer may be included together in a layer containing the HMG-CoA reductase inhibitor.

Particularly, such stabilizer may be an antioxidant, alkali metal salt, organic salt, or mixtures thereof, and more particularly may be calcium carbonate, sodium carbonate, sodium hydrogen carbonate, sodium hydroxide, magnesium oxide, magnesium carbonate, sodium citrate, calcium acetate, meglumine, triethanolamine, arginine glycine, butylated hydroxytoluene (BHT), dibutyl hydroxytoluene (DHT), butylated hydroxyanisole (BHA), sodium sulfite, sodium pyrosulfite, sodium hydrogensulfite, propyl gallate, calcium phosphate, or mixtures thereof, but not limited thereto.

As said binder, the binders conventionally used in a pharmaceutical industry may be all usable. For example, the following may be used as the binder: starch, microcrystalline cellulose, high-dispersed silica, mannitol, saccharose, lactose, polyethylene glycol, polyvinyl pyrrolidone, hydroxypropyl methylcellulose, hydroxypropyl cellulose, sodium carboxymethyl cellulose, pre-gelatinized starch, natural gum, synthetic gum, polyvinyl pyrrolidone copolymer, povidone, copovidone, gelatin, mixtures thereof or the like, but not limited thereto. A content of the binder may be appropriately selected by those skilled in the art. For example, such content may be appropriately chosen within a range of 0.0001 to 200 parts by weight per 1 part by weight of an active component.

As said disintegrant, the disintegrants conventionally used in the pharmaceutical industry may be all usable. For example, the following may be used as the disintegrant: starch or modified starch such as sodium starch glycolate, maize starch, potato starch, pre-gelatinized starch or the like; clay such as bentonite, montmorillonite, veegum or the like; celluloses such as microcrystalline cellulose, hydroxypropyl cellulose, carboxymethyl cellulose or the like; aligns such as sodium alginate, alginic acid or the like; cross-linked celluloses such as croscarmellose sodium, etc.; gums such as guar gum, xanthan gum, etc.; cross-linked polymers such as cross-linked polyvinyl pyrrolidone (crospovidone), etc.; and effervescent agents such as sodium bicarbonate, citric acid, etc. or mixtures thereof, but not limited thereto. A content of the disintegrant may be appropriately selected by those skilled in the art. For example, such content may be appropriately chosen within a range of 0.0001 to 200 parts by weight per 1 part by weight of an active component.

As said lubricant, the lubricants conventionally used in the pharmaceutical industry may be all usable. The following may be used as the lubricant: talc, stearic acid, magnesium stearate, calcium stearate, sodium lauryl sulfate, hydrogenated vegetable oil, sodium benzoate, sodium stearyl fumarate, glyceryl behenate, glyceryl monooleate, glyceryl monostearate, glyceryl palmitostearate, polyethylene glycols or mixtures thereof, magnesium lauryl sulfate, sodium benzoate, polyoxyethylene monostearate, glyceryl triacetate, sucrose monolaurate, zinc stearate, hardened vegetable oil, light liquid paraffin, paraffins, leads, etc., but not limited thereto. A content of the lubricant may be appropriately selected by those skilled in the art. For example, such content may be appropriately chosen within a range of 0.0001 to 100 parts by weight per 1 part by weight of an active component.

As said diluent, the diluents conventionally used in the pharmaceutical industry may be all usable. For example, the following may be representatively used as the diluent: lactose, microcrystalline cellulose, starch, mannitol, etc. Besides, the following may be used as the diluent: white sugar, sorbitol and inorganic salts such as dibasic calcium phosphate, tribasic calcium phosphate, aluminum silicate, calcium sulfate, etc., but not limited thereto. A content of the diluent may be appropriately selected by those skilled in the art. For example, such content may be appropriately chosen within a range of 0.0001 to 200 parts by weight per 1 part by weight of an active component.

The complex preparation according to the present invention may be formulated into a preparation for oral administration such as a tablet such as a plain tablet, coated tablet, multi-layered tablet, cored tablet or the like, a powder preparation, a granule preparation, a pellet preparation, a capsule preparation or the like.

Said powder, granule or pellet may be 1500 *µ*m or less, particularly 1000 *µ*m or less. If a size of powder, granule or pellet is more than 1500 *µ*m, a degree of mixing between two pharmacologically active components may be decreased, and thus content uniformity may be lowered.

The complex preparation of the present invention is intended for preventing or treating a cardiovascular disease, in which the cardiovascular disease includes hypertension; or all the hypertension, complications and the like of those who suffer from a so-called metabolic syndrome, which develops diabetes, obesity, hyperlipidemia, coronary artery disease, etc. in a complex way, and the cardiovascular disease also includes angina, hypertension, arteriospasm, deep vein, cerebral infarction, cardiomegaly, congestive heart failure, myocardial infarction and the like.

The present invention provides a method for preparing a complex preparation, involving steps of: (a) preparing a first active component compartment including a first active component; (b) preparing a separation membrane including a hydrophobic compound; and (c) preparing a second active component compartment including a second active component.

A preparation method of the present invention may involve steps of: preparing a first active component layer including a first active component; forming a separation membrane including a hydrophobic compound on said first active component layer; and preparing a second active component layer including a second active component on said separation membrane.

Particularly, the present invention may involve steps of: (a) preparing a plain tablet including a first active component; (b) forming a separation membrane by coating a surface of said plain tablet with a composition including a hydrophobic compound; and (c) forming a membrane including a second active component on said separation membrane.

In the present invention, said preparation method may further involve a step of forming a coating membrane. For example, said coating membrane may be formed on one of the first active component layer or the second active component layer, and may be formed on both of the first and second active component layers. Said coating membrane may include one or more coating bases generally used in the art, for example, povidone, copovidone, methyl cellulose, hydroxymethyl cellulose, hydroxyethyl cellulose, hydroxypropyl cellulose, hydroxypropyl methylcellulose, hydroxypropyl ethylcellulose, gelatin and gum, but not limited thereto.

In the preparation method of the present invention, said first active component layer may be formed by mixing a pharmaceutically acceptable carrier and the first active component, or may be prepared by coating an inactive core made of the carrier with a composition including the first active component, and said carrier may be a pharmaceutically acceptable carrier generally used in the art.

In the preparation method of the present invention, said first active component and second active component may be i) a pharmaceutically acceptable acid salt of clopidogrel, optical isomers thereof, hydrates or solvates thereof, or mixtures thereof; or ii) an HMG-CoA reductase inhibitor, pharmaceutically acceptable salts thereof, optical isomers thereof, hydrates or solvates thereof, or mixtures thereof, in which said first active component and second active component may be different from each other.

A process for preparing the complex preparation according to the present invention may be performed based on a conventional process of a pharmaceutical industry. According to the present preparation method, coating may be performed by means of a general coating method in the art, and particularly such coating may be done by means of a fluidized bed granulator. For example, said separation membrane including the hydrophobic compound may be formed by coating a carrier (core) containing said first active component with a coating solution including the hydrophobic compound by means of the fluidized bed granulator. Also, a coating membrane including said second active component may be formed by coating said hydrophobic separation membrane with a coating solution including the second active component by means of the fluidized bed granulator.

Also, the complex preparation according to the present invention may be coated by means of a tablet coating machine. For example, said separation membrane including the hydrophobic compound may be formed by coating a plain tablet containing said first active component with the coating solution including the hydrophobic compound by means of the tablet coating machine. Also, said coating membrane including the second active component may be formed by coating said hydrophobic separation membrane with the coating solution including the second active component by means of the tablet coating machine.

The preparation method of the present invention may involve steps of: (a) preparing a first particle including a first active component; (b) preparing a second particle including a second active component; and (c) forming a separation membrane containing a hydrophobic compound.

Particularly, the preparation method of the present invention may involve steps of: (a) preparing a first particle including a first active component; (b) preparing a second particle including a second active component; and (c) coating one or more of said first particle and second particle with a separation membrane containing a hydrophobic compound.

In exemplary embodiments of the present invention, said preparation method may involve steps of: (a) preparing a first particle including a first active component; (b) coating a surface of said first particle with a separation membrane containing a hydrophobic compound; and (c) mixing said first particle coated with the separation membrane and the second particle including the second active component.

In the preparation method of the present invention, said first or second particle may be formed by using a pharmaceutically acceptable carrier, in which said carrier is as described above.

In the preparation method of the present invention, said first active component and second active component are as described above.

Matters mentioned in the preparation of the present invention may be also equally applied to the preparation method, if not contradictory to each other.

### Advantageous Effects

A complex preparation according to the present invention may effectively treat a cardiovascular disease of various causes by including clopidogrel and an HMG-CoA reductase inhibitor at the same time; has excellent storage stability by preventing a decrease in the stability of the HMG-CoA reductase inhibitor caused by a proton (H+) derived from a clopidogrel acid salt; and thus may be used as a valuable complex preparation for a use in preventing or treating the cardiovascular disease.

### Brief Description of the Drawings

Fig. 1 shows a content of lactone related substances of rosuvastatin as a result of the stability test on the inventive complex preparation (tablet) under an accelerated condition for four months.
Fig. 2 shows a content of total related substances of rosuvastatin as a result of the stability test on the inventive complex preparation (tablet) under the accelerated condition for four months.
Fig. 3 shows a content of lactone related substances of rosuvastatin as a result of the stability test on the inventive complex preparation (capsule preparation) under the accelerated condition for one month.
Fig. 4 shows a content of total related substances of rosuvastatin as a result of the stability test on the inventive complex preparation (capsule preparation) under the accelerated condition for one month.
Fig. 5 shows a comparative elution rate of rosuvastatin between the inventive complex preparation and Crestor Tab. 10 mg at pH 1.2.
Fig. 6 shows a comparative elution rate of clopidogrel between the inventive complex preparation and Plavix Tab. 75 mg at pH 1.2.
Fig. 7 shows a change of rosuvastatin concentration in blood with a beagle dog after an oral administration of the inventive complex preparation and Crestor Tab. 10 mg.
Fig. 8 shows a change of clopidogrel concentration in blood with a beagle dog after an oral administration of the inventive complex preparation and Plavix Tab. 75 mg.

### Mode for Invention

The features and advantages of the present invention as well as methods for achieving them will be apparent with reference to exemplary embodiments described in detail hereinafter. However, the present invention is not limited to the exemplary embodiments disclosed hereinafter, but will be implemented in various different forms. Hereinafter, the following exemplary embodiments will be suggested for better understanding of the present invention and be provided only for the purpose of completely illustrating the scope of the present invention to those skilled in the art, and thus the present invention will be defined only by the scope of the claims thereto.

### <Example 1> Preparation of a clopidogrel plain tablet

In accordance with components and contents as shown in a following table 1, clopidogrel, L-HPC, lactose, copovidone, colloidal silicon dioxide, talc and magnesium stearate were mixed, compressed and prepared into a plain tablet. At that time, the plain tablet was made by means of a tablet press (ERWEKA APPARATEBAU).

**[Table 1]**

| Compartment | Component | Component Amount (mg/Tab.) |
|---|---|---|
| (1) Clopidogrel plain tablet | Clopidogrel hydrogensulfate | 97.9 |
| | L-HPC | 25.0 |
| | Lactose | 110.1 |
| | Copovidone | 12.0 |
| | Colloidal silicon dioxide | 2.5 |
| | Talc | 4.0 |
| | Magnesium stearate | 3.5 |
| Solid content amount | | 255.0 |

### <Examples 2 to 5> Tablet including a separation membrane containing a hydrophobic compound

In accordance with components and contents as shown in a following table 2, (2) a coating solution for a hydrophobic separation membrane containing a hydrophobic compound and (3) a coating solution containing rosuvastatin were respectively prepared, after which (1) a clopidogrel plain tablet of Example 1 was sequentially coated with (2) the coating solution for the hydrophobic separation membrane and (3) the coating solution containing rosuvastatin, such that a coating layer was prepared. At that time, coating was performed at a spray velocity of 20 g/min at an inlet temperature of 58-72°C for 180 minutes by using a tablet coating machine (FREUND HCT-30).

**[Table 2]**

| Compartment | Component | Component Amount (mg/Tab.) | | | |
|---|---|---|---|---|---|
| | | Example 2 | Example 3 | Example 4 | Example 5 |
| (1) Clopidogrel plain tablet | Example 1 | 255.0 | 255.0 | 255.0 | 255.0 |
| (2) Coating solution for hydrophobic separation membrane | Glyceryl behenate | 10.0 | | | |
| | Sodium stearyl fumarate | | 10.0 | | |
| | Carnauba wax | | | 10.0 | |
| | Lecithin | | | | 10.0 |
| | Copovidone | 10.0 | 10.0 | 10.0 | 10.0 |
| | Titanium oxide | 0.5 | 0.5 | 0.5 | 0.5 |
| | Talc | 0.5 | 0.5 | 0.5 | 0.5 |
| | Purified water (volatile) | 75.0 | 75.0 | 75.0 | 75.0 |
| | Ethanol (volatile) | 300.0 | 300.0 | 300.0 | 300.0 |
| (3) Rosuvastatin coating solution | Rosuvastatin calcium | 10.4 | 10.4 | 10.4 | 10.4 |
| | Copovidone | 59.6 | 59.6 | 59.6 | 59.6 |
| | Talc | 1.0 | 1.0 | 1.0 | 1.0 |
| | Purified water (volatile) | 70.0 | 70.0 | 70.0 | 70.0 |
| | Ethanol (volatile) | 200.0 | 200.0 | 200.0 | 200.0 |
| Solid content amount | | 347.0 | 347.0 | 347.0 | 347.0 |

### <Comparative Examples 1 to 3> Tablet without a separation membrane or tablet including a pH-dependent separation membrane

In accordance with components and contents as shown in a following table 3, (4) a coating solution for a pH-dependent separation membrane and (3) a coating solution containing rosuvastatin were respectively prepared, after which (1) the clopidogrel plain tablet of Example 1 was sequentially coated with (4) the coating solution for the pH-dependent separation membrane and (3) the rosuvastatin coating solution. However, in case of a complex preparation without a separation membrane in Comparative Example 1, coating for the separation membrane was omitted and coating was performed with (3) the coating solution containing rosuvastatin. At that time, coating was performed at a spray velocity of 20 g/min at an inlet temperature of 58-72°C for 180 minutes by using a tablet coating machine (FREUND HCT-30).

**[Table 3]**

| Compartment | Component | Component Amount (mg/Tab.) | | |
|---|---|---|---|---|
| | | Comparative Example 1 | Comparative Example 2 | Comparative Example 3 |
| (1) Clopidogrel plain tablet | Example 1 | 255.0 | 255.0 | 255.0 |
| (4) Coating solution for no separation membrane and pH-dependent separation membrane | Eudragit EPO | | 15.0 | |
| | Eudragit L100 | | | 17.0 |
| | Sodium lauryl sulfate | | 1.5 | |
| | Triethylacetate | | | 1.5 |
| | Stearic acid | | 2.0 | |
| | Talc | | 7.5 | 7.5 |
| | Purified water (volatile) | | 150.0 | 150.0 |
| (3) Rosuvastatin coating solution | Rosuvastatin calcium | 10.4 | 10.4 | 10.4 |
| | Copovidone | 59.6 | 59.6 | 59.6 |
| | Talc | 1.0 | 1.0 | 1.0 |
| | Purified water (volatile) | 70.0 | 70.0 | 70.0 |
| | Ethanol (volatile) | 200.0 | 200.0 | 200.0 |
| Solid content amount | | 321.0 | 347.0 | 347.0 |

### <Comparative Examples 4 to 6> Tablet including a hydrophilic separation membrane

In accordance with components and contents as shown in a following table 4, (5) a coating solution for a hydrophilic separation membrane without containing a hydrophobic compound and (3) a coating solution containing rosuvastatin were respectively prepared, after which (1) the clopidogrel plain tablet of Example 1 was sequentially coated with (5) the coating solution for the hydrophilic separation membrane and (3) the coating solution containing rosuvastatin. At that time, coating was performed at a spray velocity of 20 g/min at an inlet temperature of 58-72°C for 180 minutes by using a tablet coating machine (FREUND HCT-30).

**[Table 4]**

| Compartment | Component | Component Amount (mg/Tab.) | | |
|---|---|---|---|---|
| | | Comparative Example 4 | Comparative Example 5 | Comparative Example 6 |
| (1) Clopidogrel plain tablet | Example 1 | 255.0 | 255.0 | 255.0 |
| (5) Coating layer for hydrophilic separation membrane | Povidone | 20.0 | | |
| | Copovidone | | 20.0 | |
| | PVA | | | 20.0 |
| | Titanium oxide | 0.5 | 0.5 | 0.5 |
| | Talc | 0.5 | 0.5 | 0.5 |
| | Purified water (volatile) | 75.0 | 75.0 | 75.0 |
| | Ethanol (volatile) | 300.0 | 300.0 | 300.0 |
| (3) Rosuvastatin coating layer | Rosuvastatin calcium | 10.4 | 10.4 | 10.4 |
| | Copovidone | 59.6 | 59.6 | 59.6 |
| | Talc | 1.0 | 1.0 | 1.0 |
| | Purified water (volatile) | 70.0 | 70.0 | 70.0 |
| | Ethanol (volatile) | 200.0 | 200.0 | 200.0 |
| Solid content amount | | 347.0 | 347.0 | 347.0 |

### < Comparative Examples 7 and 8> Single tablet

A commercially available rosuvastatin preparation (Crestor Tab. 10mg, AstraZeneca, Comparative Example 7) and a clopidogrel preparation (Plavix Tab. 75mg, Sanofi-Aventis, Comparative Example 8) were purchased and used.

### < Example 6> Capsule preparation including a hydrophobic separation membrane

In accordance with components and contents as shown in a following table 5, (3) a coating solution containing rosuvastatin and (2) a coating solution for a hydrophobic separation membrane containing a hydrophobic compound were respectively prepared, after which an inactive pellet (seed pellet) was sequentially coated with (3) the coating solution containing rosuvastatin and (2) the coating solution for the hydrophobic separation membrane, then mixed with clopidogrel hydrogensulfate powder, and then filled into a capsule. At that time, pellet coating was performed at a spray velocity of 20-50 mg/min at an inlet temperature of 35-65°C for 10 hours by using a fluidized bed coating machine (FREUND GPC-G1) in a bottom mode.

### < Comparative Example 9> Capsule preparation without a separation membrane

In accordance with components and contents as shown in a following table 5, coating for the hydrophobic separation membrane was omitted and an inactive pellet (seed pellet) was coated with (3) a coating solution containing rosuvastatin, then mixed with clopidogrel hydrogensulfate powder, and then filled into a capsule. At that time, pellet coating was performed at a spray velocity of 20-50 mg/min at an inlet temperature of 35-65°C for 10 hours by using a fluidized bed coating machine (FREUND GPC-G1) in a bottom mode.

**[Table 5]**

| Compartment | Component | Component Amount (mg/Cap.) | |
|---|---|---|---|
| | | Example 6 | Comparative Example 9 |
| Inactive pellet | Microcrystalline cellulose pellet | 150.0 | 150.0 |
| (3) Rosuvastatin coating solution | Rosuvastatin calcium | 10.4 | 10.4 |
| | Copovidone | 20.6 | 20.6 |
| | Talc | 4.0 | 4.0 |
| | Purified water (volatile) | 80.0 | 80.0 |
| | Ethanol (volatile) | 170.0 | 170.0 |
| (2) Coating solution for hydrophobic separation membrane | Opaglos (Lecithin=6.1% w/w) | 50.0 (Lecithin=3.05 mg/Tab.) | - |
| | Povidone K30 | 50.0 | - |
| | Purified water (volatile) | 900.0 | - |
| | Ethanol (volatile) | 100.0 | - |
| Clopidogrel | Clopidogrel hydrogensulfate | 97.9 | 97.9 |
| Capsule | | Capsule no. 1 | Capsule no. 1 |
| Solid content amount (excluding the capsule) | | 382.9 | 282.9 |

### < Experimental Example 1> Disintegration test

In case of Examples 2 to 5 and Comparative Examples 1 to 6 above, a disintegration test was performed at pH 1.2 with a 1^{st} test solution for disintegration test methods of the Korean Pharmacopoeia. The disintegration test method is as follows and results thereof are as shown in a following table 6.

### [Test Method]

1. Basis for disintegration test: Disintegration test of general test methods in the 10^{th} revision of the Korean Pharmacopoeia
2. Test method: Each of six samples was inserted into a glass tube of a testing machine, and the testing machine was put into operation for two hours with a test solution set at 37 ± 2°C.
3. Test solution: A 1^{st} test solution (pH 1.2) for disintegration test methods of the Korean Pharmacopoeia
4. Analysis method: A point of time, at which properties of all six samples completely disappear, was measured.

### [Test Results]

**[Table 6]**

| | Disintegration time |
|---|---|
| Example 2 | 15 min 17 sec |
| Example 3 | 13 min 48 sec |
| Example 4 | 25 min 12 sec |
| Example 5 | 21 min 30 sec |
| Comparative Example 1 | 10 min 26 sec |
| Comparative Example 2 | 23 min 31 sec |
| Comparative Example 3 | Timeout |
| Comparative Example 4 | 10 min 15 sec |
| Comparative Example 5 | 10 min 21 sec |
| Comparative Example 6 | 10 min 50 sec |

As shown in the table 6 above, in case of Examples 2 to 5 and Comparative Examples 1, 2 and 4 to 6 according to the present invention, an disintegration time was 30 minutes or so at pH 1.2 similar to a gastrointestinal tract, and thus it was identified that a drug may be easily dissolved in the gastrointestinal tract.

On the other hand, in case of Comparative Example 3, disintegration was not done at pH 1.2 within two hours, and thus an elution of an active component was delayed. Eudragit L100, which was a separation membrane base of Comparative Example 3 above, may be dissolved at pH 6.0 or more, and thus it may be identified that Eudragit L100 is not desirable as a separation membrane of clopidogrel and the HMG-CoA inhibitor, which requires a release in the gastrointestinal tract.

### < Experimental Examples 2> Stability test

In case of Examples 2 to 4 and Comparative Examples 1 to 6, each of tablets was packed into an HDPE bottle along with silica gel, then stored under an accelerated condition, then taken out in zero (initial), two and four months later, and then evaluated by measuring an amount of rosuvastatine lactone related substances and total related substances, which were representative acid decomposition products of rosuvastatine out of the HMG-CoA reductase inhibitor. The stability test method is as follows and results thereof are as shown in a following table 7 and Figs. 1 and 2.

### [Test Method]

1. Basis for stability test: Items of the accelerated test in the Ministry of Food and Drug Safety Notification No. 2014-59
2. Test method: Each of 30 tablets was packed into a 100 mL HDPE bottle along with silica gel, then stored in a stability chamber under an accelerated condition (40±2°C, relative humidity of 75±5%), then opened in zero (initial), two and four months later, and then analyzed. At that time, the test was finished, if an amount of total related substances exceeds 5%.
3. Analysis method: An amount of lactone related substances and total related substances, which were representative acid decomposition products, was analyzed according to a method for analyzing related substances of rosuvastatin.

### [Test Results]

**[Table 7]**

| | Accelerated test period (month) | Lactone related substance (%) | Total related substance (%) | Remark (Separation membrane form) |
|---|---|---|---|---|
| Comparative Example 1 | 0 (initial) | 0.050 | 0.304 | No separation membrane |
| | 2 | 4.970 | 5.130 | |
| Comparative Example 2 | 0 (initial) | 0.050 | 0.304 | pH-dependent separation membrane |
| | 2 | 7.723 | 7.923 | |
| Comparative Example 3 | 0 (initial) | 0.051 | 0.385 | |
| | 2 | 6.180 | 6.378 | |
| Comparative Example 4 | 0 (initial) | 0.051 | 0.307 | Hydrophilic separation membrane without |
| | 2 | 5.841 | 6.115 | |
| Comparative Example 5 | 0 (initial) | 0.055 | 0.366 | containing a hydrophobic compound |
| | 2 | 3.007 | 3.661 | |
| | 4 | 7.858 | 8.994 | |
| Comparative Example 6 | 0 (initial) | 0.062 | 0.491 | |
| | 2 | 9.139 | 10.168 | |
| Example 2 | 0 (initial) | 0.050 | 0.377 | Separation membrane containing a hydrophobic compound |
| | 2 | 0.000 | 0.555 | |
| | 4 | 0.013 | 0.688 | |
| Example 3 | 0 (initial) | 0.050 | 0.377 | |
| | 2 | 0.000 | 0.475 | |
| | 4 | 0.249 | 0.914 | |
| Example 4 | 0 (initial) | 0.050 | 0.377 | |
| | 2 | 0.000 | 0.460 | |
| | 4 | 0.166 | 0.864 | |

As shown in the table 7 above and Fig. 1, it was identified for Examples 2 to 4 of the present invention that a growth rate of lactone related substances was as very low as less than 1% for four months under the accelerated condition.

On contrary, it was identified for Comparative Examples 1 to 6 that the lactone related substances were greatly increased by 3% or more within two months under the accelerated condition.

In particular, in case of Comparative Examples 2 to 5, the lactone related substances were more greatly increased. That's because protons (H⁺) were accumulated in a pH-dependent separation membrane and a hydrophilic water-soluble separation membrane without containing a hydrophobic compound, and thus the lactone related substances with low stability in acid were greatly increased.

### <Experimental Example 3> Stability test on a capsule preparation

In case of Example 6 and Comparative Example 9, each of capsule preparations was individually packed with Alu-Alu blister, then stored under an accelerated condition, then taken out in zero (initial) and one month later, and then evaluated by measuring an amount of rosuvastatine lactone related substances and total related substances, which were representative acid decomposition products of rosuvastatine out of the HMG-CoA reductase inhibitor. The stability test method is as follows and results thereof are as shown in a following table 8 and Figs. 3 and 4.

### [Test Method]

1. Basis for stability test: Items of the accelerated test in the Ministry of Food and Drug Safety Notification No. 2014-59
2. Test method: Each of capsule preparations was packed with Alu-Alu blister, then stored in a stability chamber under an accelerated condition (40±2°C, relative humidity of 75±5%), then opened in zero (initial) and one month after, and then analyzed.
3. Analysis method: An amount of lactone related substances and total related substances, which were representative acid decomposition products, was analyzed according to a method for analyzing related substances of rosuvastatin.

### [Test Results]

**[Table 8]**

| | Accelerated test period (month) | Lactone related substance (%) | Total related substance (%) | Remark (Separation membrane form) |
|---|---|---|---|---|
| Example 6 | 0 (initial) | 0.113 | 0.332 | Hydrophobic separation membrane |
| | 1 | 0.192 | 0.309 | |
| Comparative Example 9 | 0 (initial) | 0.145 | 0.368 | No separation membrane |
| | 1 | 0.512 | 0.710 | |

As shown in the table 8 above and Fig. 3, it was identified for Example 6 of the present invention that an amount of increase in lactone related substances was as very low as less than 0.08% under the accelerated condition for one month.

On contrary, it was identified for Comparative Example 9 that an amount of increase in lactone related substances was 0.36% or more under the accelerated condition as early as one month, and such great increase was at least 4.5 times more than Example 6. Accordingly, it was also identified that an increase in lactone related substances might be inhibited even in a capsule preparation filled with a pellet coated with a hydrophobic separation membrane.

### <Experimental Example 4> Elution test

A comparative elution test was performed with each of four tablets of Example 2 and Comparative Examples 7 and 8 above. A method for the comparative elution test is as follows and results thereof are as shown in a following table 9 and Figs. 5 and 6.

### [Test Method]

1. Basis for elution test: Elution test method of general test methods in the 10^{th} revision of the Korean Pharmacopoeia
2. Test method: Paddle method, 37±0.5°C, 50 revolutions/min, n=4
3. Test solution: 900 ml of 1^{st} test solution for disintegration test methods of the Korean Pharmacopoeia (pH 1.2)
4. Analysis method: Method for analyzing an elution (content) of rosuvastatin and clopidogrel

### [Test Results]

**[Table 9]**

| | | | 0 min | 5 min | 10 min | 15 min | 30 min | 45 min | 60 min | 90 min | 120 min |
|---|---|---|---|---|---|---|---|---|---|---|---|
| Rosuvastatin elution rate (%) | Example 2 | Average | 0.0 | 16.5 | 47.3 | 71.3 | 87.2 | 90.9 | 93.2 | 96.0 | 96.3 |
| | | S.D. | 0.0 | 5.5 | 9.5 | 7.3 | 4.9 | 4.0 | 4.8 | 3.5 | 3.1 |
| | Comparative Example 7 | Average | 0.0 | 49.9 | 77.5 | 90.1 | 97.2 | 98.7 | 98.5 | 99.3 | 99.1 |
| | | S.D. | 0.0 | 2.3 | 3.0 | 3.1 | 2.7 | 2.2 | 1.8 | 1.3 | 1.3 |
| Clopidogrel elution rate | Example 2 | Average | 0.0 | 0.0 | 2.1 | 7.8 | 46.8 | 80.7 | 95.3 | 98.1 | 98.7 |
| | | S.D. | 0.0 | 0.0 | 0.5 | 2.3 | 8.4 | 7.6 | 5.1 | 2.0 | 1.1 |
| (%) | Comparative Example 8 | Average | 0.0 | 22.4 | 46.4 | 67.9 | 86.1 | 91.6 | 93.9 | 94.5 | 95.4 |
| | | S.D. | 0.0 | 6.2 | 8.5 | 9.9 | 8.9 | 6.1 | 5.4 | 5.2 | 2.9 |

As shown in the table 9 and Fig. 5, in case of both Example 2 and Comparative Example 7 of the present invention, a drug of rosuvastatin was eluted within 30 minutes and both preparations were eluted 80% or more within 30 minutes, which was shorter than about two hours, that is, a general drug retention time in the gastrointestinal tract. However, in case of Example 2 of the present invention, an elution rate of rosuvastatin was faster than Comparative Example 7 under the condition above, seemingly because a coated rosuvastatin of Example 2 may be released into eluate faster than rosuvastatin of the tablet of Comparative Example 7.

Meanwhile, as shown in the table 9 and Fig. 6, in case of both Example 2 and Comparative Example 8 of the present invention, clopidogrel was eluted 80% or more within 60 minutes, which was shorter than about two hours, that is, a general drug retention time in the gastrointestinal tract. However, in case of Example 2, an elution rate of clopidogrel was slower than Comparative Example 8 under the condition above, because clopidogrel is eluted after a complete dissolution of a rosuvastatin coating layer and the separation membrane due to structural properties of the preparation of Example 2.

### < Experimental Example 5> Blood concentration test

With regard to Example 2 (test group) and Comparative Examples 7 and 8 (control group) above, each of preparations was orally administered into a beagle dog, and then a blood concentration test was performed on the test group and the control group according to the present invention.

Meanwhile, with regard to the test group and the control group, each group of four beagle dogs was subjected to a cross-matching test (2 x 2) twice at a drug-free interval of two weeks. A detailed experimental condition is as shown in a following table 10. Also, with regard to the control group and the test group, the results of the blood concentration test on rosuvastatin and clopidogrel are as shown in a table 11 and Figs. 7 and 8.

**[Table 10]**

| Title | Oral PK test on a beagle dog for a hydrophobic separation membrane base and a single/combined preparation with rosuvastatin/clopidogrel |
|---|---|
| Test design | The present test was designed as follows. |
| | - Control group: Two tablets of rosuvastatin 10 mg (Crestor Tab. 10 mg) |
| | + Two tablets of clopidogrel 75 mg (Plavix Tab. 75 mg) |
| | - Test group: Two tablets of the hydrophobic separation membrane prepared in Example 2 |
| | |
| | Each of the control group and the test group was subjected to a cross-matching test twice at a drug-free interval of two weeks. |
| Evaluation method | Evaluation of drug concentration in blood: |
| | After administration of a test drug, a concentration of rosuvastatin and clopidogrel in blood was measured by means of LC/MS/MS, and a difference between groups was compared and evaluated with following items set as parameters. |
| | - Area under the blood concentration-time curve up to a last measurable point of blood collection: AUCₗₐₛₜ |
| | - Area under the blood concentration-time curve calculated by means of extrapolation from a last measurable point of blood collection to infinite time: AUC_{inf} |
| | - Max. blood concentration: Cₘₐₓ |
| | - Time to reach Max. blood concentration: Tₘₐₓ |
| | - Drug half-life: T_{1/2} |

### [Test Results]

**[Table 11]**

| PK parameter | Rosuvastatin | | Clopidogrel | |
|---|---|---|---|---|
| | Control group | Test group | Control group | Test group |
| Cₘₐₓ (ng/ml) | 138.563 ±24.745 | 121.908 ±47.555 | 249.14 ±90.056 | 223.618 ±107.886 |
| Tₘₐₓ (hr) | 1.6±0.3 | 1.4±0.8 | 1.4±0.5 | 1.4±0.6 |
| AUCₗₐₛₜ (ng/ml) | 462.246 ±106.647 | 422.990 ±213.641 | 435.725 ±196.644 | 364.373 ±137-952 |
| AUC_{inf} (ng/ml) | 552.449 ±138.648 | 513.212 ±244.548 | 448.219 ±200.370 | 381.545 ±142.895 |
| t_{½} (hr) | 2.3±0.6 | 2.5±0.5 | 1.2±0.5 | 1.6±0.8 |

According to the table 11 and Figs. 7 and 8, in case of the test group (Example 2) and the control group (Comparative Examples 7 and 8) of the present invention, there was no significant difference in AUC, Cmax, Tmax and T_{½} values of rosuvastatin and clopidogrel.

In case of Experimental Example 3 above, there was a slight difference in elution rates of Example 2 and Comparative Examples 7 and 8. However, in case of the PK test on the beagle dog, there was no significant difference in AUC, Cmax, Tmax and T_{½} values. Thus, it might be identified that there is no great change in pharmacokinetic parameters of the drug through an in vivo experiment.

In the complex preparation containing an HMG-CoA reductase inhibitor and clopidogrel of the present invention, the preparation coated with a separation membrane containing a hydrophobic substance of the present invention may provide the complex preparation with excellent storage stability without any PK change of an active component in vivo, and thus may be utilized as a therapeutic agent for a cardiovascular disease with excellent stability.

## Claims

1. A complex preparation, comprising:
a pharmaceutically acceptable acid salt of clopidogrel, optical isomers thereof, hydrates or solvates thereof, or mixtures thereof;
an HMG-CoA reductase inhibitor, pharmaceutically acceptable salts thereof, optical isomers thereof, hydrates or solvates thereof, or mixtures thereof; and
a separation membrane containing a hydrophobic compound.

2. The complex preparation according to claim 1, wherein said complex preparation comprises a first active component-containing layer including a first active component, a separation membrane layer containing a hydrophobic compound, and a second active component-containing layer including a second active component;
wherein said first and second active components are different from each other; and
wherein said first and second active components are one of i) a pharmaceutically acceptable acid salt of clopidogrel, optical isomers thereof, hydrates or solvates thereof, or mixtures thereof; or ii) an HMG-CoA reductase inhibitor, pharmaceutically acceptable salts thereof, optical isomers thereof, hydrates or solvates thereof, or mixtures thereof.

3. The complex preparation according to claim 2, wherein said complex preparation comprises:
a) a first layer containing said first active component;
b) a second layer, i.e., a separation membrane, formed on said first layer and containing a hydrophobic compound; and
c) a third layer formed on said second layer and including said second active component.

4. The complex preparation according to claim 2 or 3, wherein said separation membrane prevents said first active component and second active component from being in contact with each other.

5. The complex preparation according to claim 1, wherein said complex preparation comprises a first particle including a first active component, a second particle including a second active component, and a separation membrane containing a hydrophobic compound;
wherein the separation membrane containing the hydrophobic compound separates the first particle and the second particle from each other, and thus prevents said first active component and second active component from being in contact with each other; and
wherein said first and second active components are one of i) a pharmaceutically acceptable acid salt of clopidogrel, optical isomers thereof, hydrates or solvates thereof, or mixtures thereof; or ii) an HMG-CoA reductase inhibitor, pharmaceutically acceptable salts thereof, optical isomers thereof, hydrates or solvates thereof, or mixtures thereof.

6. The complex preparation according to claim 5, wherein said separation membrane is formed on one or more of a first or second particle.

7. The complex preparation according to claim 5, wherein said complex preparation comprises:
a) an inactive pellet including a pharmaceutically acceptable carrier;
b) a first particle including a first active component formed on said pellet;
c) a separation membrane containing a hydrophobic compound formed on said first particle; and
d) a second particle physically separated from the first particle and including a second active component.

8. The complex preparation according to claim 7, wherein said second particle consists of an active component only.

9. The complex preparation according to claim 7, wherein said second particle comprises an active component and a pharmaceutically acceptable carrier.

10. The complex preparation according to claim 1, wherein said complex preparation comprises said first active component, said second active component, and a particle including a separation membrane containing a hydrophobic compound, which prevents said first active component and said second active component from being in contact with each other; and
wherein said particle comprises:
a first layer including said first active component;
a second layer including said second active component; and
a separation membrane present between said first layer and second layer.

11. The complex preparation according to claim 1 or 5, wherein said pharmaceutically acceptable salt of clopidogrel is one or more selected from the group consisting of hydrogensulfate, hydrochloride, napadisilate, besylate, bromate, taurocholate and acetate.

12. The complex preparation according to claim 1 or 5, wherein said HMG-CoA reductase inhibitor is one or more selected from the group consisting of rosuvastatin, atorvastatin, simvastatin, lovastatin, mevastatin, pravastatin, fluvastatin, cerivastatin, pitavastatin, bervastatin, dalvastatin, glenvastatin, salts thereof and isomers thereof.

13. The complex preparation according to claim 1 or 5, wherein said HMG-CoA reductase inhibitor is one or more selected from the group consisting of rosuvastatin, atorvastatin, pravastatin and fluvastatin, salts thereof and isomers thereof.

14. The complex preparation according to claim 1 or 5, wherein said clopidogrel, pharmaceutically acceptable salts thereof, hydrates or solvates thereof, optical isomers thereof, or mixtures thereof are included in an amount of 75 to 300 mg.

15. The complex preparation according to claim 1 or 5, wherein said separation membrane comprises a hydrophobic compound.

16. The complex preparation according to claim 1 or 5, wherein said separation membrane comprises a hydrophobic compound in an amount of 0.01 to 0.8 parts by weight per 1 part by weight of the separation membrane.

17. The complex preparation according to claim 16, wherein said hydrophobic compound is one or more selected from the group consisting of glyceryl palmitostearate, glyceryl stearate, glyceryl behenate, cetyl palmitate, lecithin, glyceryl monooleate, stearic acid, cetostearyl alcohol, cetyl alcohol, stearyl alcohol, carnauba wax, cera and microcrystalline wax.

18. The complex preparation according to claim 16, wherein said hydrophobic compound is one or more selected from the group consisting of glyceryl behenate, sodium stearyl fumarate, carnauba wax and lecithin.

19. The complex preparation according to claim 1 or 5, wherein said separation membrane comprises a hydrophilic compound in an amount of 0.2 to 0.99 parts by weight per 1 part by weight of the separation membrane.

20. The complex preparation according to claim 19, wherein said hydrophilic compound is one or more selected from the group consisting of hydroxypropyl methylcellulose, hydroxypropyl cellulose, hydroxymethyl cellulose, hydroxyethyl cellulose, methyl cellulose, sodium carboxymethyl cellulose, hydroxypropyl methylcellulose acetate succinate, hydroxyethyl methylcellulose, guar gum, locust bean gum, tragacanth, carrageenan, acacia gum, arabic gum, gellan gum, xanthan gum, gelatin, casein, zein, polyvinyl alcohol, polyvinyl pyrrolidone, copolyvidone, polyvinyl acetal dietylamino acetate, poly(butylmethacrylate-(2-dimethylaminoethyl)methacrylate-methylmethacrylate)copolymer, poly(methacrylic acid-methylmethacrylate)copolymer, poly(methacrylic acid-ethylacrylate)copolymer, polyethylene glycol, polyethylene oxide and carbomer.

21. The complex preparation according to claim 1 or 5, wherein said separation membrane comprises a hydrophobic compound and a hydrophilic compound in an amount of 1 : 10 to 3 : 1 parts by weight.

22. The complex preparation according to claim 1 or 5, wherein said complex preparation further comprises one or more additives selected from the group consisting of a stabilizer, binder, disintegrant, lubricant, diluent, coating agent, pH-adjusting agent, dissolution aid and surfactant.

23. The complex preparation according to claim 22, wherein said stabilizer is an antioxidant, alkali metal salt, organic salt or mixtures thereof.

24. The complex preparation according to claim 23, wherein said stabilizer is calcium carbonate, sodium carbonate, sodium hydrogen carbonate, magnesium oxide, magnesium carbonate, sodium citrate, meglumine, triethanolamine, arginine, glycine, butylated hydroxytoluene (BHT), dibutyl hydroxytoluene (DHT), butylated hydroxylanisole (BHA), sodium sulfite, sodium pyrosulfite, sodium hydrogensulfite, propyl gallate, calcium phosphate or mixtures thereto.

25. The complex preparation according to claim 1 or 5, wherein said preparation is a plain tablet, coated tablet, multi-layered tablet, cored tablet, powder preparation, granule preparation, pellet preparation or capsule preparation.

26. The complex preparation according to claim 1 or 5, wherein said complex preparation is intended for preventing or treating a cardiovascular disease selected from the group consisting of angina, hypertension, arteriospasm, deep vein, cerebral infarction, cardiomegaly, congestive heart failure and myocardial infarction.

27. A method for preparing a complex preparation, wherein the method comprises steps of:
(a) preparing a plain tablet including a first active component;
(b) forming a separation membrane by coating a surface of said plain tablet with a composition including a hydrophobic compound; and
(c) forming a membrane including a second active component on said separation membrane,
wherein said first and second active components are different from each other; and
wherein said first and second active components are one of i) clopidogrel, pharmaceutically acceptable salts thereof, hydrates or solvates thereof, optical isomers thereof, or mixtures thereof; or ii) an HMG-CoA reductase inhibitor, pharmaceutically acceptable salts thereof, hydrates or solvates thereof, optical isomers thereof, or mixtures thereof.
